# EUROPÄISCHE PATENTSCHRIFT

(11) **EP 0 432 504 B1**
(45) Veröffentlichungstag und Bekanntmachung des Hinweises auf die Patenterteilung: **08.06.1994**
(21) Anmeldenummer: 90121827.1
(22) Anmeldetag: 14.11.1990
(51) Int. Cl.: C07C 229/28, C07C 227/00, C07C 255/46

(54) **Verfahren zur Herstellung von 1-(Aminomethyl)cyclohexanessigsäure**
Process for preparing 1-(aminomethyl)cyclohexane acetic acid
Procédé pour la préparation de l'acide (aminométhyl)-1 cyclohexane-acétique

(30) Priorität: 16.11.1989 CH 4128/89
(43) Veröffentlichungstag der Anmeldung: 19.06.1991
(73) Patentinhaber: LONZA AG, CH-4002 Basel (CH)
(72) Erfinder: Mettler, Hans Peter, Dr., Brig-Glis (Kanton Wallis) (CH); Previdoli, Felix, Dr., Brig (Kanton Wallis) (CH)
(74) Vertreter: Weinhold, Peter, Dr.

(56) Entgegenhaltungen:
- EP-A- 0 358 092
- DE-A- 2 044 216
- DRUGS OF THE FUTURE, Band IX, Nr. 6, 1984 "Gabapentin" Seiten 418,419

## Beschreibung

Die Erfindung betrifft ein neues Verfahren zur Herstellung von 1-(Aminomethyl)cyclohexanessigsäure sowie (1-Cyanocyclohexyl)essigsäureester als neue Zwischenprodukte im erfindungsgemässen Verfahren.

Die 1-(Aminomethyl)cyclohexanessigsäure wird unter dem Namen Gabapentin in der Medizin als Anticonvulsivum eingesetzt. Gabapentin, seine Anwendung und Herstellung werden in Drugs of the Future, Vol.9 Nr.6, 1984, S.418-419, sowie in den US-Patenten 4 024 175 und 4 152 326 beschrieben. Diese bekannte Herstellung ist sehr aufwendig und verläuft über 7 bis 8, zum Teil technisch heikle Stufen.

Es bestand daher die Aufgabe, ein Verfahren zu finden, das die genannten Nachteile ausschliesst.
Die Aufgabe konnte gelöst werden mit einem Verfahren gemäss Patentanspruch 1.

Ausgangsprodukt im erfindungsgemässen Verfahren sind die (1-Cyanocyclohexyl)malonsäuredialkylester der allgemeinen Formel 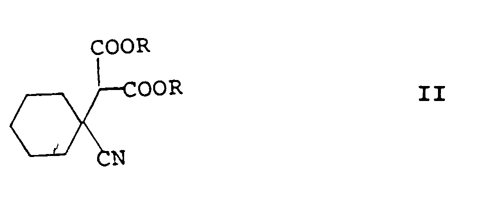
worin R = Alkyl mit 1 bis 4 C-Atomen bedeutet. Diese Verbindungen sind in der EP-A1-0 358 092 beschrieben und auf einfache Weise aus Cyclohexanon zugänglich.

Für das erfindungsgemässe Verfahren wurden vorzugsweise die Methyl- oder Ethylester eingesetzt.

In der ersten Verfahrensstufe wird das Ausgangsprodukt zu einem (1-Cyanocyclohexyl)essigsäurealkylester der allgemeinen Formel 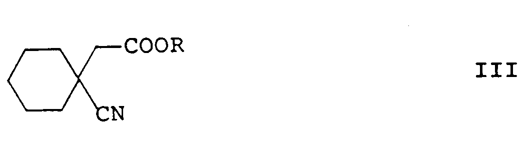
worin R die genannte Bedeutung hat, decarbalkoxyliert. Diese Verbindungen sind bisher nicht beschrieben.

Die Decarbalkoxylierung kann nach literaturbekannten Methoden, z.B. nach Krapcho et al, Synthesis 1982, S.805, oder nach Aneya et al, Tetrahedron Letters 1983, Vol.24, S.4641 durchgeführt werden.
Danach wird zweckmässig in Borsäureanhydrid oder in dipolar aprotischen Lösungsmitteln, wie z.B. Dimethylsulfoxid, in Verbindung mit Wasser als Reaktionsmedium bei Temperaturen zwischen 100 und 250°C gearbeitet. Wahlweise kann die Reaktion in Gegenwart von Alkali- oder Erdalkalisalzen, wie Alkali- oder Erdalkalichloride, -cyanide oder -acetate, durchgeführt werden.

Der resultierende (1-Cyanocyclohexyl)essigsäurealkylester wird zweckmässig isoliert und durch Destillation gereinigt.

Gemäss Patentanspruch 3 besteht alternativ die Möglichkeit, die (1-Cyanocyclohexyl)essigsäurealkylester durch Alkoholyse von (1-Cyanocyclohexyl)acetonitril der allgemeinen Formel 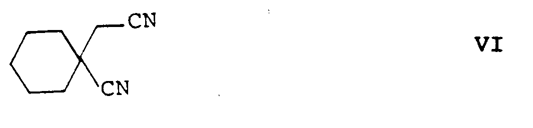
herzustellen. Das (1-Cyanocyclohexyl)acetonitril ist dabei gemäss New et al, Synthesis 1983, S.388, aus dem entsprechenden Cyclohexylidenmalonsäureester zugänglich.

Die Alkoholyse wird mit einem niederen Alkohol, vorzugsweise mit Methanol oder Ethanol, in Gegenwart einer Mineralsäure aus der Reihe Chlorwasserstoff, Bromwasserstoff oder wasserfreie Schwefelsäure durchgeführt.
Bezogen auf das Nitril wird sowohl der Alkohol als auch die Mineralsäure zweckmässig in Mengen von 1 bis 100 Aequivalenten eingesetzt.

Die Reaktionstemperatur liegt zweckmässig zwischen -20 und 50°C, der Druck zwischen 1 und 10 bar.

Gegebenenfalls kann zum Alkohol als Reaktand ein zusätzliches aprotisches Lösungsmittel, wie z.B. ein aliphatischer oder aromatischer Kohlenwasserstoff, ein Ether, Ester, oder ein halogenierter Kohlenwasserstoff eingesetzt werden.

Das bei der Alkoholyse auftretende Zwischenprodukt (Imidat) wird nicht isoliert, sondern direkt mit Wasser, vorzugsweise im Ueberschuss, bei Temperaturen zwischen -20 bis 100°C zum (1-Cyanocyclohexyl)essigsäurealkylester hydrolysiert.

In der Folgestufe erfolgt die Umesterung des Alkylesters mit einem Benzylalkohol der allgemeinen Formel 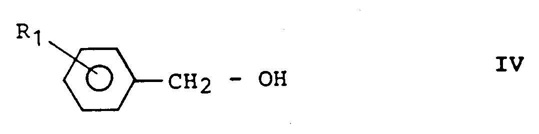
worin R₁ = H oder eine Alkoxygruppe eine Nitrogruppe oder Halogen bedeutet, in Gegenwart eines Katalysators zu einem (1-Cyanocyclohexyl)essigsäurebenzylester der allgemeinen Formel 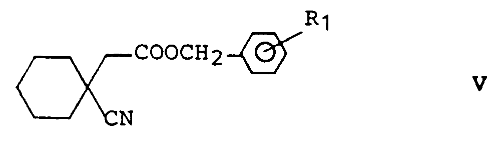
worin R₁ die genannte Bedeutung hat.

Diese Verbindungen sind bisher nicht beschrieben.

Die Umesterung wird vorzugsweise mit Benzylalkohol in Gegenwart einer Base als Katalysator durchgeführt.
Geeignete Basen sind die Cyanide wie z.B. Kaliumcyanid, Alkoholate wie z.B. Natriummethylat oder Kalium-tert.butylat, oder tertiäre Amine wie z.B. Triethylamin oder N,N-Dimethylaminopyridin.

Der Katalyt gelangt zweckmässig in Mengen zwischen 0,01 und 10 mol%, vorzugsweise zwischen 0,2 und 3 mol% zum Einsatz.

Vorteilhaft wird in Gegenwart eines aprotischen Lösungsmittels, wie Diethylether oder Tetrahydrofuran, oder eines aromatischen oder aliphatischen Kohlenwasserstoffs, wie Toluol oder Hexan, gearbeitet.

Die Reaktionstemperatur für die Umesterung liegt zweckmässig zwischen 0°C und der Siedetemperatur des eingesetzten Benzylalkohols.
Das Reaktionsprodukt wird zweckmässig isoliert und durch Destillation gereinigt.

Gemäss Patentanspruch 2 besteht alternativ die Möglichkeit, den (1-Cyanocyclohexyl)essigsäurebenzylester durch Alkoholyse von (1-Cyanocyclohexyl)acetonitril der allgemeinen Formel 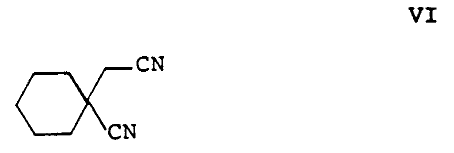
mit einem Benzylalkohol der allgemeinen Formel 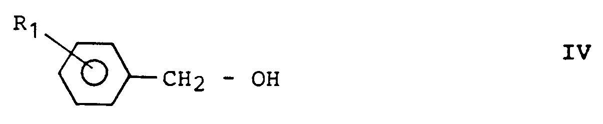
worin R₁ = H oder eine Alkoxygruppe, eine Nitrogruppe oder Halogen bedeutet, vorzugsweise mit Benzylalkohol, in Gegenwart einer Mineralsäure aus der Reihe Chlorwasserstoff, Bromwasserstoff oder wasserfreie Schwefelsäure herzustellen.

Sowohl der Benzylalkohol als auch die Mineralsäure werden zweckmässig in Mengen von 1 bis 100 Aequivalenten, bezogen auf das Nitril, eingesetzt.

Die Reaktionstemperatur liegt zweckmässig zwischen -20 und 50°C, der Druck zwischen 1 und 10 bar.

Gegebenenfalls kann zum Alkohol als Reaktand ein zusätzliches aprotisches Lösungsmittel, wie z.B. ein aliphatischer oder aromatischer Kohlenwasserstoff, ein Ether, Ester, oder ein halogenierter Kohlenwasserstoff eingesetzt werden.

Das bei der Alkoholyse auftretende Zwischenprodukt (Imidat) wird nicht isoliert, sondern direkt mit Wasser, vorzugsweise im Ueberschuss, bei Temperaturen zwischen -20 bis 100°C zum (1-Cyanocyclohexyl)essigsäurebenzylester hydrolysiert.

In der letzten Stufe erfolgt die katalytische Hydrierung des Benzylesters mit Wasserstoff zum gewünschten Endprodukt, der 1-(Aminomethyl)cyclohexanessigsäure.

Als Hydrierkatalysator können Edelmetallkatalysatoren, wie Platin-, Palladium-, Rhodium-, Rutheniumkatalysatoren, gegebenenfalls aufgebracht auf inerten Trägermaterialien wie z.B. Aktivkohle oder Aluminiumoxid, oder Raney-Katalysatoren wie z.B. Raney-Nickel oder Raney-Cobalt-Katalysatoren, oder (Edel) Metalloxiden wie z.B. Nickeloxid oder Platinoxid verwendet werden.

Zweckmässig bewegt sich die Menge Katalysator zwischen 1 und 50 Gew.%, bezogen auf den eingesetzten Benzylester.

Vorteilhaft wird in Gegenwart eines geeigneten Lösungsmittels, wie eines niederen Alkohols, z.B. Ethanol, Methanol, einer Carbonsäure, z.B. Essigsäure, eines Esters, z.B. Ethylacetat, oder eines Ethers oder Alkohols in Verbindung mit Ammoniak, hydriert.

Der Druck bewegt sich zweckmässig im Bereich von 1 bis 100 bar, vorzugsweise zwischen 2 und 10 bar, die Temperatur zwischen 0 und 100°C. Die optimale Temperatur ist weitgehend abhängig vom eingesetzten Katalysator.

Das gewünschte Endprodukt fällt bereits in hoher Reinheit an, kann aber gegebenenfalls durch Umkristallisation weiter gereinigt werden.

### Beispiel 1

### Herstellung von (1-Cyanocyclohexyl)essigsäureethylester

26,9 g (100 mol) (1-Cyanocyclohexyl)malonsäurediethylester, 4,3 g (100 mol) Lithiumchlorid und 3,6 g (200 mol) Wasser wurden in 300 ml Dimethylsulfoxid während 22 h auf 150°C erhitzt. Anschliessend wurde abgekühlt, mit 700 ml Wasser vermischt und mit 1000 ml Pentan extrahiert.
Durch Destillation der organischen Phase erhielt man 14,4 g (1-Cyanocyclohexyl)essigsäureethylester.
Ausbeute: 74%, bezogen auf eingesetzten (1-Cyanocyclohexyl) malonsäurediethylester.
Sdp.: 125-130°C/2-4 mbar

| Elementaranalyse für C₁₁H₁₇NO₂(195,3): | | | |
|---|---|---|---|
| ber. | C 67,7% | H 8,8% | N 7,2% |
| gef. | C 67,7% | H 8,7% | N 7,0% |

¹H-NMR: (DMSO-D₆, 300 MHz) δ
1,20 (t, 3H)
1,10-1,25 (m, 1H)
1,34-1,56 (m, 4H)
1,61-1,77 (m, 3H)
1,93-2,03 (m, 2H)
2,69 (s, 2H)
4,11 (q, 2H)

### Beispiel 2

### Herstellung von (1-Cyanocyclohexyl)essigsäurebenzylester

401 mg (2 mmol) (1-Cyanocyclohexyl)essigsäureethylester, 1,09 g (10 mmol) Benzylalkohol und 6 mg (0,1 mmol) Kaliumcyanid wurden in 5 ml Toluol während 24 h zum Rückfluss erhitzt. Anschliessend wurde die Lösung mit 25 ml Wasser gewaschen, vom Lösungsmittel befreit und am Hochvakuum destilliert. Man erhielt 350 mg (1-Cyanocyclohexyl)essigsäurebenzylester.
Ausbeute: 68%, bezogen auf eingesetzten (1-Cyanocyclohexyl) essigsäureethylester.
Sdp.: 148-152°C/0,1-0,2 mbar

| Elementaranalyse für C₁₆H₁₉NO₂(257,3): | | | |
|---|---|---|---|
| ber. | C 74,7% | H 7,4% | 5,4% |
| gef. | C 74,9% | H 7,4% | 5,5% |

¹H-NMR: (CDCl₃, 300 MHz) δ
1,13-1,27 (m, 1H)
1,28-1,42 (m, 2H)
1,59-1,80 (m, 5H)
2,04-2,12 (m, 2H)
2,59 (s, 2H)
5,12 (s, 2H)
7,30-7,41 (m, 5H)

### Beispiel 3

### Herstellung von 1-(Aminomethyl)cyclohexanessigsäure

1,0 g (3,8 mmol) (1-Cyanocyclohexyl)essigsäurebenzylester wurde in 20 ml Methanol gelöst, mit 0,2 g Rh/C 5% versetzt und bei 10 bar Wasserstoffdruck hydriert. Nach 23 h bei Raumtemperatur wurde die Suspension filtriert, das Filtrat auf 3 ml eingeengt, mit 25 ml Ethanol versetzt, auf 4 ml aufkonzentriert und kühlgestellt. Das ausgefallene Produkt wurde abfiltriert, mit Ethanol gewaschen und getrocknet. Man erhielt 0,18 g Gabapentin.
Ausbeute: 27%, bezogen auf eingesetzten (1-Cyanocyclohexyl)essigsäurebenzylester.
Smp.: 148-151°C
¹H-NMR: (CD₃OD, 300 MHz) δ
1,30-1,67 (m, 10H)
2,47 (s, 2H)
2,89 (s, 2H)

### Beispiel 4

### Herstellung von (1-Cyanocyclohexyl)essigsäurebenzylester aus (1-Cyanocyclohexyl)acetonitril

1,52 g (10 mmol) (1-Cyanocyclohexyl)acetonitril und 13,1 g (120 mmol) Benzylalkohol wurden in 20 ml Toluol bei 0°C mit HCl-Gas gesättigt. Nach 22 h wurde mit 25 ml Wasser und 100 ml Diethylether versetzt, 30 min gut gerührt und filtriert. Die organische Phase wurde abgetrennt und eingeengt.
Man erhielt 13 g Produkt, das gemäss Gaschromatographie 2,22% (1-Cyanocyclohexyl)essigsäurebenzylester enthielt.
Ausbeute: 11%, bezogen auf eingesetztes (1-Cyanocyclohexyl)acetonitril.

### Beispiel 5

### Herstellung von (1-Cyanocyclohexyl)essigsäureethylester aus (1-Cyanocyclohexyl)acetonitril

(1-Cyanocyclohexyl)acetonitril (7,60 g, 50 mmol) wurden in 30 ml Ethanol suspendiert und im Autoklav bei 0°C mit HCl-Gas gesättigt (3 bar). Nach 22 Stunden wurde entspannt, 30 Min. evakuiert (18 mbar), mit 150 ml Wasser vermischt und 3 Stunden bei 10°C gerührt. Anschliessend wurde am Rotationsverdampfer auf 168 g aufkonzentriert und mit 50 ml Ethylacetat extrahiert. Aus der organischen Phase liessen sich mittels Destillation 4,04 g (1-Cyanocyclohexyl)essigsäureethylester isolieren.
Ausbeute: 41% (bezogen auf eingesetztes (1-Cyanocyclohexyl)acetonitril)

## Patentansprüche (Patentansprüche für folgende(n) Vertragsstaat(en): AT, BE, CH, DE, DK, FR, GB, IT, LI, NL, SE)

1. Verfahren zur Herstellung von 1-(Aminomethyl)cyclohexanessigsäure der Formel 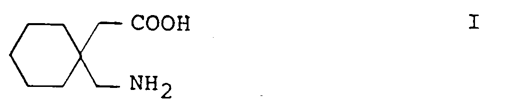 dadurch gekennzeichnet, dass in der ersten Stufe ein (1-Cyanocyclohexyl)malonsäuredialkylester der allgemeinen Formel 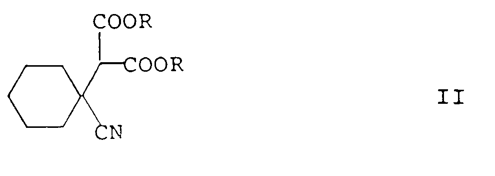 worin R = Alkyl mit 1 bis 4 C-Atomen bedeutet, zum entsprechenden (1-Cyanocyclohexyl)essigsäurealkylester der allgemeinen Formel 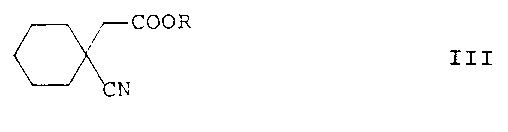 worin R die genannte Bedeutung hat, decarbalkoxyliert wird, weiter in der zweiten Stufe mit einem Benzylalkohol der allgemeinen Formel 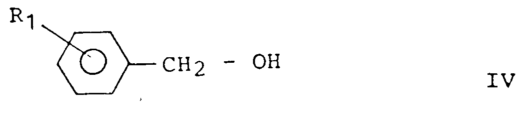 worin R₁ = H, eine Alkoxygruppe, eine Nitrogruppe oder Halogen bedeutet, in Gegenwart eines basischen Katalysators zu einem (1-Cyanocyclohexyl)essigsäurebenzylester der allgemeinen Formel 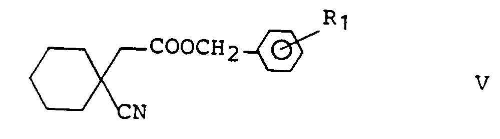 worin R₁ die genannte Bedeutung hat, umgeestert wird und schliesslich in Gegenwart eines Hydrierkatalysators mit Wasserstoff zum Endprodukt hydriert wird.

2. Verfahren zur Herstellung von 1-(Aminomethyl)cyclohexanessigsäure der Formel 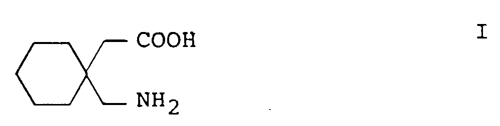 dadurch gekennzeichnet, daß (1-Cyanocyclohexyl)acetonitril der Formel 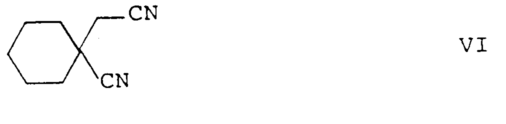 mit einem Benzylalkohol der allgemeinen Formel 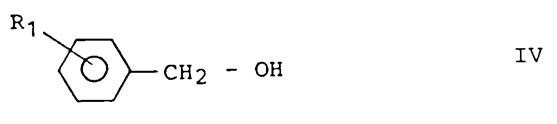 worin R₁ die genannte Bedeutung hat, in Gegenwart einer Mineralsäure umgesetzt und das Reaktionsprodukt anschließend mit Wasser hydrolysiert wird, worauf der entstandene (1-Cyanocyclohexyl)essigsäurebenzylester in Gegenwart eines Hydrierungskatalysators mit Wasserstoff zum Endprodukt hydriert wird.

3. Verfahren zur Herstellung von 1-(Aminomethyl)cyclohexanessigsäure der Formel 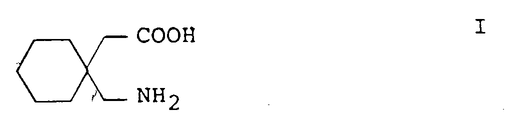 dadurch gekennzeichnet, daß (1-Cyanocyclohexyl)acetonitril der Formel 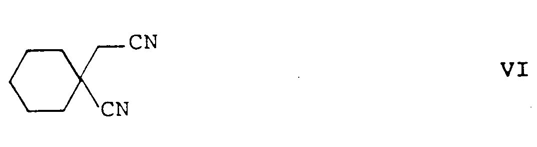 mit einem aliphatischen C₁-C₄-Alkohol in Gegenwart einer Mineralsäure umgesetzt, das entstandene Reaktionsprodukt anschließend mit Wasser hydrolysiert wird, worauf der entstandene (1-Cyanocyclohexyl)essigsäurealkylester in Gegenwart eines Hydrierungskatalysators mit Wasserstoff zum Endprodukt hydriert wird.

4. Verfahren nach Patentanspruch 1, dadurch gekennzeichnet, dass die Decarbalkoxylierung bei Temperaturen zwischen 100 und 250°C in Gegenwart von Borsäureanhydrid oder dipolar aprotischen Lösungsmitteln in Verbindung mit Wasser durchgeführt wird.

5. Verfahren nach Patentanspruch 4, dadurch gekennzeichnet, dass in Gegenwart von Alkali- oder Erdalkalisalzen gearbeitet wird.

6. Verfahren nach Patentanspruch 1, dadurch gekennzeichnet, dass als basischer Katalysator für die Umesterung ein Alkalicyanid, ein Alkalialkoholat oder ein tertiäres Amin in einer Menge von 0,01 bis 10 mol% verwendet wird.

7. Verfahren nach Patentanspruch 1 oder 6, dadurch gekennzeichnet, dass die Umesterung in Gegenwart eines Lösungsmittels bei Temperaturen zwischen 0°C und der Siedetemperatur des eingesetzten Benzylalkohols durchgeführt wird.

8. Verfahren nach Patentanspruch 1, 2 oder 3, dadurch gekennzeichnet, daß als Hydrierkatalysator ein Platin-, Palladium-, Ruthenium-, Rhodiumkatalysator, gegebenenfalls aufgebracht auf einen inerten Träger, ein Raney-Katalysator oder ein Metalloxidkatalysator eingesetzt wird.

9. Verfahren nach Patentanspruch 1, 2, 3 oder 8, dadurch gekennzeichnet, daß der Katalysator in Mengen von 1 bis 50 Gew.-%, bezogen auf den (1-Cyanocyclohexyl)essigsäurebenzylester verwendet wird.

10. Verfahren nach Patentanspruch 1, 2, 3, 8 oder 9, dadurch gekennzeichnet, daß die Hydrierung bie einem Druck zwischen 1 und 100 bar und bei Temperaturen zwischen 0 und 100°C durchgeführt wird.

11. Verfahren nach Patentanspruch 1, 2, 3, 8, 9 oder 10, dadurch gekennzeichnet, daß die Hydrierung in Gegenwart eines Lösungsmittels durchgeführt wird.

12. Verfahren nach Patentanspruch 2, dadurch gekennzeichnet, dass als Mineralsäure Chlorwasserstoff, Bromwasserstoff oder wasserfreie Schwefelsäure verwendet wird.

13. Verfahren nach Patentansprüchen 2 oder 12, dadurch gekennzeichnet, dass bei einem Druck zwischen 1 und 10 bar und bei Temperaturen zwischen -20 und 50°C gearbeitet wird.

14. Verfahren nach Patentanspruch 2, 12 oder 13, dadurch gekennzeichnet, dass in Gegenwart eines Lösungsmittels gearbeitet wird.

15. Verfahren nach Patentanspruch 2,12,13 oder 14, dadurch gekennzeichnet, dass die Hydrolyse mit Wasser bei Temperaturen zwischen -20 und 100°C erfolgt.

16. Verfahren nach Patentanspruch 3, dadurch gekennzeichnet, dass als Mineralsäure Chlorwasserstoff, Bromwasserstoff oder wasserfreie Schwefelsäure verwendet wird.

17. Verfahren nach Patentansprüchen 3 oder 16, dadurch gekennzeichnet, dass bei einem Druck zwischen 1 und 10 bar und bei Temperaturen zwischen -20 und 50°C gearbeitet wird.

18. Verfahren nach Patentansprüchen 3, 16 oder 17, dadurch gekennzeichnet, dass als niederer aliphatischer Alkohol Methanol oder Ethanol eingesetzt wird.

19. Verfahren nach Patentanspruch 3, 16, 17 oder 18, dadurch gekennzeichnet, dass in Gegenwart eines Lösungsmittels gearbeitet wird.

20. Verfahren nach Patentanspruch 3,16,17,18 oder 19, dadurch gekennzeichnet, dass die Hydrolyse mit Wasser bei Temperaturen zwischen -20 und 100°C erfolgt.

21. (1-Cyanocyclohexyl)essigsäureester der allgemeinen Formel 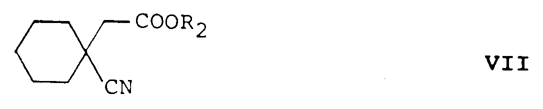 worin R₂ = Alkyl mit 1 bis 4 C-Atomen oder ein Benzylrest der allgemeinen Formel 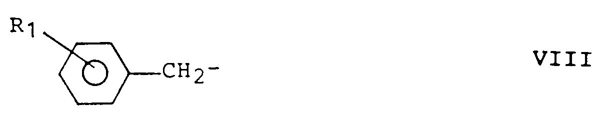 worin R₁ = H, eine Alkoxygruppe, eine Nitrogruppe oder Halogen bedeutet.

## Patentansprüche (Patentansprüche für folgende(n) Vertragsstaat(en): ES)

1. Verfahren zur Herstellung von 1-(Aminomethyl)cyclohexanessigsäure der Formel 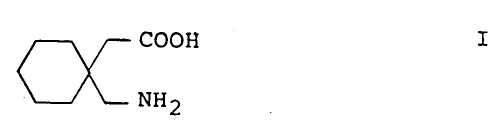 dadurch gekennzeichnet, dass in der ersten Stufe ein (1-Cyanocyclohexyl)malonsäuredialkylester der allgemeinen Formel 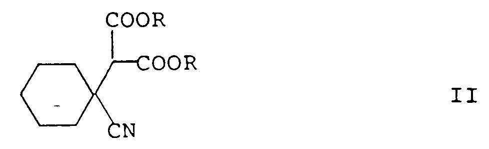 worin R = Alkyl mit 1 bis 4 C-Atomen bedeutet, zum entsprechenden (1-Cyanocyclohexyl)essigsäurealkylester der allgemeinen Formel 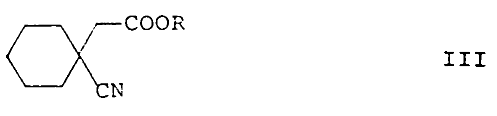 worin R die genannte Bedeutung hat, decarbalkoxyliert wird, weiter in der zweiten Stufe mit einem Benzylalkohol der allgemeinen Formel

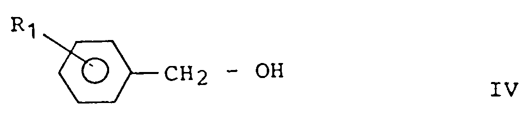

2. Verfahren zur Herstellung von 1-(Aminomethyl)cyclohexanessigsäure der Formel 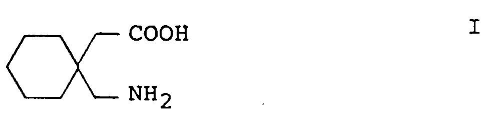 dadurch gekennzeichnet, daß (1-Cyanocyclohexyl)acetonitril der Formel 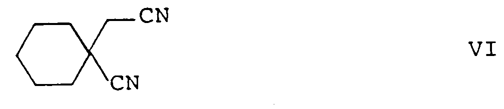 mit einem Benzylalkohol der allgemeinen Formel 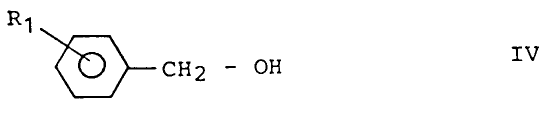 worin R₁ die genannte Bedeutung hat, in Gegenwart einer Mineralsäure umgesetzt und das Reaktionsprodukt anschließend mit Wasser hydrolysiert wird, worauf der entstandene (1-Cyanocyclohexyl)essigsäurebenzylester in Gegenwart eines Hydrierungskatalysators mit Wasserstoff zum Endprodukt hydriert wird.

3. Verfahren zur Herstellung von 1-(Aminomethyl)cyclohexanessigsäure der Formel 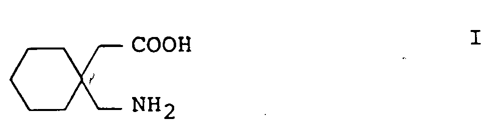 dadurch gekennzeichnet, daß (1-Cyanocyclohexyl)acetonitril der Formel 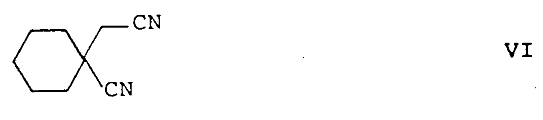 mit einem aliphatischen C₁-C₄-Alkohol in Gegenwart einer Mineralsäure umgesetzt, das entstandene Reaktionsprodukt anschließend mit Wasser hydrolysiert wird, worauf der entstandene (1-Cyanocyclohexyl)essigsäurealkylester in Gegenwart eines Hydrierungskatalysators mit Wasserstoff zum Endprodukt hydriert wird.

4. Verfahren nach Patentanspruch 1, dadurch gekennzeichnet, dass die Decarbalkoxylierung bei Temperaturen zwischen 100 und 250°C in Gegenwart von Borsäureanhydrid oder dipolar aprotischen Lösungsmitteln in Verbindung mit Wasser durchgeführt wird.

5. Verfahren nach Patentanspruch 4, dadurch gekennzeichnet, dass in Gegenwart von Alkali- oder Erdalkalisalzen gearbeitet wird.

6. Verfahren nach Patentanspruch 1, dadurch gekennzeichnet, dass als basischer Katalysator für die Umesterung ein Alkalicyanid, ein Alkalialkoholat oder ein tertiäres Amin in einer Menge von 0,01 bis 10 mol% verwendet wird.

7. Verfahren nach Patentanspruch 1 oder 6, dadurch gekennzeichnet, dass die Umesterung in Gegenwart eines Lösungsmittels bei Temperaturen zwischen 0°C und der Siedetemperatur des eingesetzten Benzylalkohols durchgeführt wird.

8. Verfahren nach Patentanspruch 1, 2 oder 3, dadurch gekennzeichnet, daß als Hydrierkatalysator ein Platin-, Palladium-, Ruthenium-, Rhodiumkatalysator, gegebenenfalls aufgebracht auf einen inerten Träger, ein Raney-Katalysator oder ein Metalloxidkatalysator eingesetzt wird.

9. Verfahren nach Patentanspruch 1, 2, 3 oder 8, dadurch gekennzeichnet, daß der Katalysator in Mengen von 1 bis 50 Gew.-%, bezogen auf den (1-Cyanocyclohexyl)essigsäurebenzylester verwendet wird.

10. Verfahren nach Patentanspruch 1, 2, 3, 8 oder 9, dadurch gekennzeichnet, daß die Hydrierung bie einem Druck zwischen 1 und 100 bar und bei Temperaturen zwischen 0 und 100°C durchgeführt wird.

11. Verfahren nach Patentanspruch 1, 2, 3, 8, 9 oder 10, dadurch gekennzeichnet, daß die Hydrierung in Gegenwart eines Lösungsmittels durchgeführt wird.

12. Verfahren nach Patentanspruch 2, dadurch gekennzeichnet, dass als Mineralsäure Chlorwasserstoff, Bromwasserstoff oder wasserfreie Schwefelsäure verwendet wird.

13. Verfahren nach Patentansprüchen 2 oder 12, dadurch gekennzeichnet, dass bei einem Druck zwischen 1 und 10 bar und bei Temperaturen zwischen -20 und 50°C gearbeitet wird.

14. Verfahren nach Patentanspruch 2, 12 oder 13, dadurch gekennzeichnet, dass in Gegenwart eines Lösungsmittels gearbeitet wird.

15. Verfahren nach Patentanspruch 2,12,13 oder 14, dadurch gekennzeichnet, dass die Hydrolyse mit Wasser bei Temperaturen zwischen -20 und 100°C erfolgt.

16. Verfahren nach Patentanspruch 3, dadurch gekennzeichnet, dass als Mineralsäure Chlorwasserstoff, Bromwasserstoff oder wasserfreie Schwefelsäure verwendet wird.

17. Verfahren nach Patentansprüchen 3 oder 16, dadurch gekennzeichnet, dass bei einem Druck zwischen 1 und 10 bar und bei Temperaturen zwischen -20 und 50°C gearbeitet wird.

18. Verfahren nach Patentansprüchen 3, 16 oder 17, dadurch gekennzeichnet, dass als niederer aliphatischer Alkohol Methanol oder Ethanol eingesetzt wird.

19. Verfahren nach Patentanspruch 3, 16, 17 oder 18, dadurch gekennzeichnet, dass in Gegenwart eines Lösungsmittels gearbeitet wird.

20. Verfahren nach Patentanspruch 3,16,17,18 oder 19, dadurch gekennzeichnet, dass die Hydrolyse mit Wasser bei Temperaturen zwischen -20 und 100°C erfolgt.

## Claims (Claims for the following Contracting State(s): AT, BE, CH, DE, DK, FR, GB, IT, LI, NL, SE)

1. A process for preparing 1-(aminomethyl) cyclohexane acetic acid of the formula: 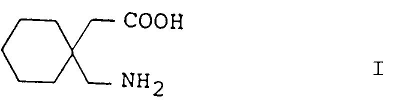 characterized in that, in a first step, (1-cyanocyclohexyl) malonic acid dialkyl ester of the general formula: 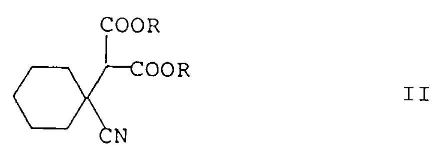 wherein R is alkyl having 1 to 4 carbon atoms, is decarbalkoxylated to the respective (1-cyanocyclohexyl) acetic acid alkyl ester of the general formula: 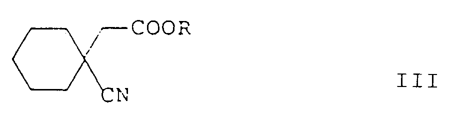 wherein R has the above meaning; is then, in a second step, transesterified with benzyl alcohol of the general formula: 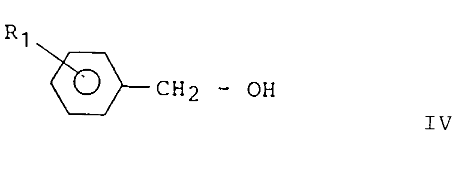 wherein R₁ is H, an alkoxy group, a nitro group or halogen, in the presence of a basic catalyst to a (1-cyanocyclohexyl) acetic acid benzyl ester of the general formula: 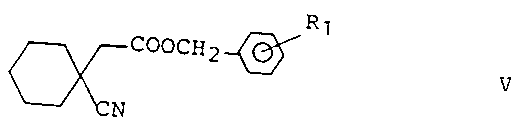 wherein R₁ has the above meaning; and is subsequently hydrogenated with hydrogen in the presence of a hydrogenation catalyst to form the final product.

2. A process for preparing 1-(aminomethyl) cyclohexane acetic acid of the formula: 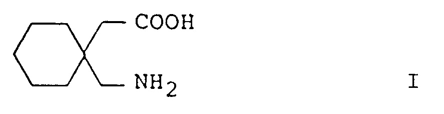 characterized in that (1-cyanocyclohexyl) acetonitrile of the formula: 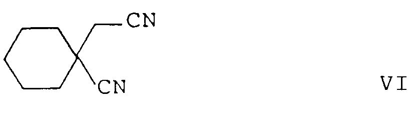 is reacted with a benzyl alcohol of the general formula: 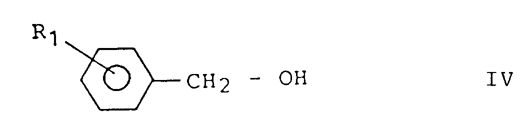 wherein R₁ has the above meaning, in the presence of a mineral acid , followed by hydrolysis of the reaction product with water, whereupon the resulting (1-cyanocyclohexyl) acetic acid benzyl ester is hydrogenated with hydrogen in the presence of a hydrogenation catalyst to form the final product.

3. A process for preparing 1-(aminomethyl) cyclohexane acetic acid of the formula: 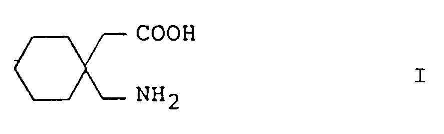 characterized by reacting (1-cyanocyclohexyl) acetonitrile of the formula: 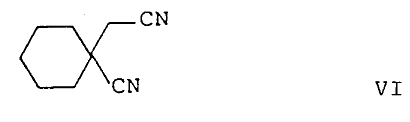 with an aliphatic C₁-C₄ alcohol in the presence of a mineral acid, hydrolizing the resulting reaction product with water, and subsequently hydrogenating the resulting (1-cyanocyclohexyl) acetic acid alkyl ester with hydrogen in the presence of a hydrogenation catalyst.

4. The process according to Claim 1, characterized in that decarbalkoxylation is conducted at temperatures between 100 and 250°C in the presence of boric anhydride or dipolar aprotic solvents in combination with water.

5. The process according to Claim 4, characterized by working in the presence of salts of alkali metals or alkaline earth metals.

6. The process according to Claim 1, characterized by using as a basic catalyst for transesterification an alkali cyanide, alkali alcoholate or a tertiary amine in an amount of from 0.01 to 10 mole-%.

7. The process according to Claim 1 or 6, characterized by conducting transesterification in the presence of a solvent at temperatures between 0°C and the boiling temperature of the benzyl alcohol used.

8. The process according to Claim 1, 2 or 3, characterized by using as a hydrogenation catalyst a platinum, palladium, ruthenium or rhodium catalyst, optionally applied to an inert substrate, a Raney catalyst or a metal oxide catalyst.

9. The process according to Claim 1, 2, 3 or 8, characterized by using the catalyst in amounts of from 1 to 50% by weight based upon the (1-cyanocyclohexyl) acetic acid benzyl ester.

10. The process according to Claim 1, 2, 3, 8 or 9, characterized by conducting hydrogenation at a pressure between 1 and 100 bar and at temperatures of from 0°C to 100°C.

11. The process accoding to Claims 1, 2, 3, 8, 9 or 10, characterized by conducting hydrogenation in the presence of a solvent.

12. The process according to Claim 2, characterized by using as a mineral acid hydrogen chloride, hydrogen bromide or anhydrous sulfuric acid.

13. The process according to Claim 2 or 12, characterized by working at a pressure between 1 and 10 bar and at temperatures of from -20°C to 50°C.

14. The process according to Claim 2, 12 or 13, characterized by working in the presence of a solvent.

15. The process according to Claim 2, 12, 13 or 14, characterized by hydrolysis with water taking place at temperatures between -20°C and 100°C.

16. The process according to Claim 3, characterized by using as a mineral acid hydrogen chloride, hydrogen bromide or anhydrous sulfuric acid.

17. The process according to Claim 3 or 16, characterized by working at a pressure between 1 and 10 bar and at temperatures of from -20°C to 50°C.

18. The process according to Claim 3, 16 or 17, characterized by using as a lower aliphatic alcohol methanol or ethanol.

19. The process according to Claim 3, 16, 17 or 18, characterized by working in the presence of a solvent.

20. The process according to Claim 3, 16, 17, 18 or 19, characterized by hydrolysis with water taking place at temperatures between -20°C and 100°C.

21. (1-Cyanocyclohexyl) acetic acid ester of the general formula: 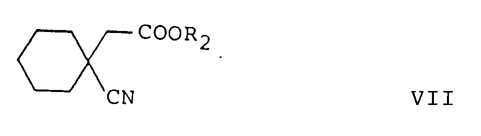 wherein R₂ is alkyl having 1 to 4 carbon atoms or a benzyl moiety of the general formula: 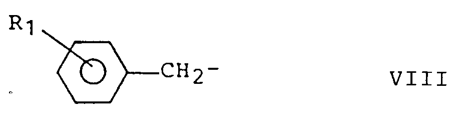 wherein R₁ is H, an alkoxy group, a nitro group or halogen.

## Claims (Claims for the following Contracting State(s): ES)

1. A process for preparing 1-(aminomethyl) cyclohexane acetic acid of the formula: 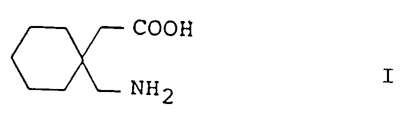 characterized in that, in a first step, (1-cyanocyclohexyl) malonic acid dialkyl ester of the general formula: 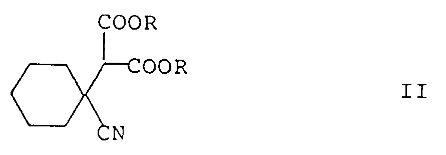 wherein R is alkyl having 1 to 4 carbon atoms, is decarbalkoxylated to the respective (1-cyanocyclohexyl) acetic acid alkyl ester of the general formula: 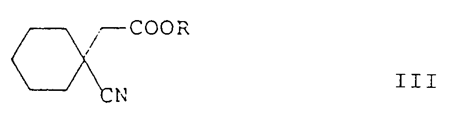 wherein R has the above meaning; is then, in a second step, transesterified with benzyl alcohol of the general formula: 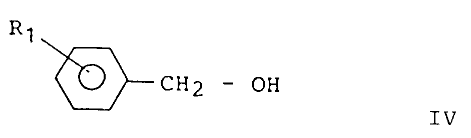 wherein R₁ is H, an alkoxy group, a nitro group or halogen, in the presence of a basic catalyst to a (1-cyanocyclohexyl) acetic acid benzyl ester of the general formula: 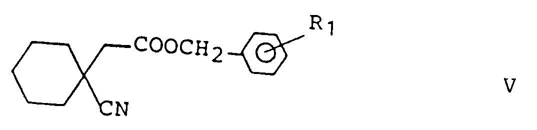 wherein R₁ has the above meaning; and is subsequently hydrogenated with hydrogen in the presence of a hydrogenation catalyst to form the final product.

2. A process for preparing 1-(aminomethyl) cyclohexane acetic acid of the formula: 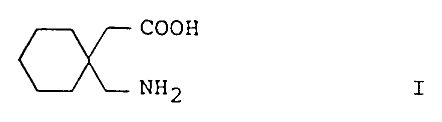 characterized in that (1-cyanocyclohexyl) acetonitrile of the formula: 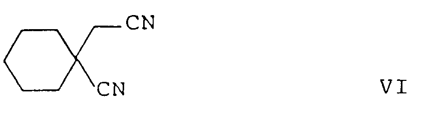 is reacted with a benzyl alcohol of the general formula: 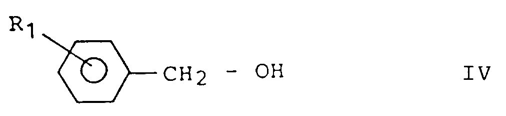 wherein R₁ has the above meaning, in the presence of a mineral acid , followed by hydrolysis of the reaction product with water, whereupon the resulting (1-cyanocyclohexyl) acetic acid benzyl ester is hydrogenated with hydrogen in the presence of a hydrogenation catalyst to form the final product.

3. A process for preparing 1-(aminomethyl) cyclohexane acetic acid of the formula: 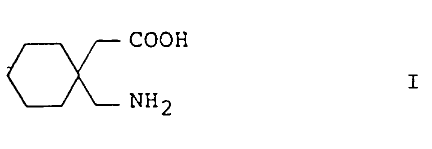 characterized by reacting (1-cyanocyclohexyl) acetonitrile of the formula: 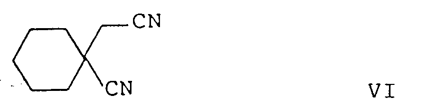 with an aliphatic C₁-C₄ alcohol in the presence of a mineral acid, hydrolizing the resulting reaction product with water, and subsequently hydrogenating the resulting (1-cyanocyclohexyl) acetic acid alkyl ester with hydrogen in the presence of a hydrogenation catalyst.

4. The process according to Claim 1, characterized in that decarbalkoxylation is conducted at temperatures between 100 and 250°C in the presence of boric anhydride or dipolar aprotic solvents in combination with water.

5. The process according to Claim 4, characterized by working in the presence of salts of alkali metals or alkaline earth metals.

6. The process according to Claim 1, characterized by using as a basic catalyst for transesterification an alkali cyanide, alkali alcoholate or a tertiary amine in an amount of from 0.01 to 10 mole-%.

7. The process according to Claim 1 or 6, characterized by conducting transesterification in the presence of a solvent at temperatures between 0°C and the boiling temperature of the benzyl alcohol used.

8. The process according to Claim 1, 2 or 3, characterized by using as a hydrogenation catalyst a platinum, palladium, ruthenium or rhodium catalyst, optionally applied to an inert substrate, a Raney catalyst or a metal oxide catalyst.

9. The process according to Claim 1, 2, 3 or 8, characterized by using the catalyst in amounts of from 1 to 50% by weight based upon the (1-cyanocyclohexyl) acetic acid benzyl ester.

10. The process according to Claim 1, 2, 3, 8 or 9, characterized by conducting hydrogenation at a pressure between 1 and 100 bar and at temperatures of from 0°C to 100°C.

11. The process accoding to Claims 1, 2, 3, 8, 9 or 10, characterized by conducting hydrogenation in the presence of a solvent.

12. The process according to Claim 2, characterized by using as a mineral acid hydrogen chloride, hydrogen bromide or anhydrous sulfuric acid.

13. The process according to Claim 2 or 12, characterized by working at a pressure between 1 and 10 bar and at temperatures of from -20°C to 50°C.

14. The process according to Claim 2, 12 or 13, characterized by working in the presence of a solvent.

15. The process according to Claim 2, 12, 13 or 14, characterized by hydrolysis with water taking place at temperatures between -20°C and 100°C.

16. The process according to Claim 3, characterized by using as a mineral acid hydrogen chloride, hydrogen bromide or anhydrous sulfuric acid.

17. The process according to Claim 3 or 16, characterized by working at a pressure between 1 and 10 bar and at temperatures of from -20°C to 50°C.

18. The process according to Claim 3, 16 or 17, characterized by using as a lower aliphatic alcohol methanol or ethanol.

19. The process according to Claim 3, 16, 17 or 18, characterized by working in the presence of a solvent.

20. The process according to Claim 3, 16, 17, 18 or 19, characterized by hydrolysis with water taking place at temperatures between -20°C and 100°C.

## Revendications (Revendications pour l'(les) Etat(s) contractant(s) suivant(s): AT, BE, CH, DE, DK, FR, GB, IT, LI, NL, SE)

1. Procédé pour la préparation de l'acide acétique de 1-(aminométhyl)cyclohexane de la formule 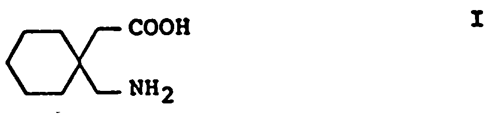 caractérisé en ce que, dans la première étape, un dialkylester de l'acide (1-cyanocyclohexyl)malonique de la formule générale 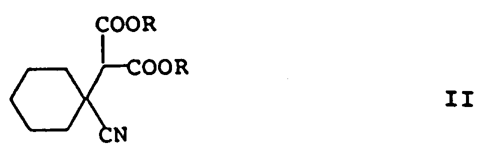 dans laquelle R signifie alkyle avec 1 jusqu'à 4 atomes de carbone, est décarbalcoxylé en l'alkylester de l'acide (1-cyanocyclohexyl)acétique correspondant de la formule générale III, 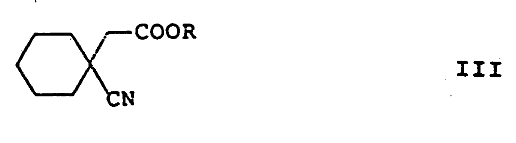 dans laquelle R a la signification indiquée, puis est transestérifié dans la deuxième étape avec un alcool de benzyle de la formule générale 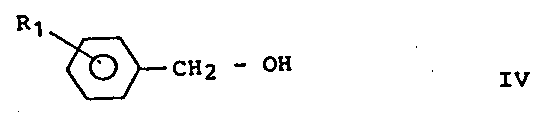 dans laquelle R₁ = H signifie un groupe alcoxy, un groupe nitro ou un halogène, en présence d'un catalyseur basique en un benzylester de l'acide (1-cyanocyclohexyl)acétique de la formule générale 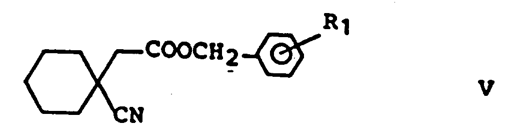 dans laquelle R₁ a la signification indiquée, puis il est hydrogéné en présence d'un catalyseur d'hydrogénation avec de l'hydrogène pour donner le produit final.

2. Procédé pour la préparation de l'acide acétique 1-(aminométhyl)cyclohexane de la formule 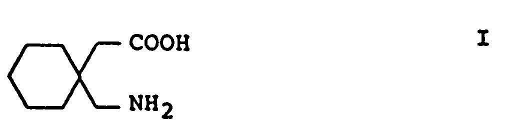 caractérisé en ce que le (1-cyanocyclohexyl)acétonitrile de la formule 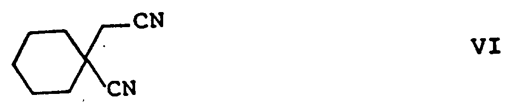 est mis en réaction avec un alcool de benzyle de la formule générale 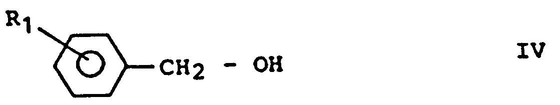 dans laquelle R₁ a la signification indiquée, en présence d'un acide minéral, et le produit réactionnel est ensuite hydrolysé avec de l'eau, après quoi le benzylester de l'acide (1-cyanocyclohexyl)acétique obtenu est hydrogéné en présence d'un catalyseur d'hydrogénation avec de l'hydrogène pour donner le produit final.

3. Procédé pour la préparation de l'acide acétique de 1-(aminométhyl)cyclohexane de la formule 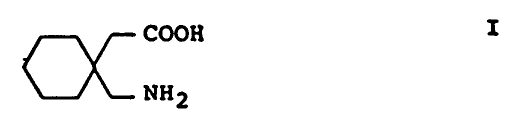 caractérisé en ce que le (1-cyanocyclohexyl)acétonitrile de la formule 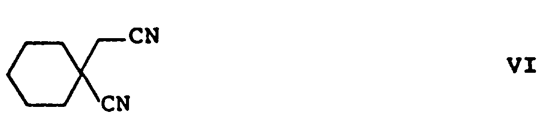 est mis en réaction avec un alcool aliphatique en C1-C4, en présence d'un acide minéral, le produit réactionnel obtenu est ensuite hydrolysé avec de l'eau, après quoi l'alkylester obtenu de l'acide (1-cyanocyclohexyl)acétique est hydrogéné en présence d'un catalyseur d'hydrogénation avec de l'hydrogène pour donner le produit final.

4. Procédé selon la revendication 1, caractérisé en ce que la décarbalcoxylation est effectuée à des températures entre 100 et 250°C, en présence d'anhydride de l'acide borique ou de solvants aprotiques dipolaires en combinaison avec de l'eau.

5. Procédé selon la revendication 4, caractérisé en ce que l'on travaille en présence de sels alcalins ou alcalino-terreux.

6. Procédé selon la revendication 1, caractérisé en ce qu'en tant que catalyseur basique pour la transestérification, on utilise un cyanure alcalin, un alcoolat alcalin ou une amine tertiaire dans une quantité de 0,01 jusqu'à 10 % en moles.

7. Procédé selon la revendication 1 ou 6, caractérisé en ce que la transestérification s'effectue en présence d'un solvant à des températures entre 0°C et la température d'ébullition de l'alcool de benzyle mis en oeuvre.

8. Procédé selon la revendication 1, 2 ou 3, caractérisé en ce qu'en tant que catalyseur d'hydrogénation, on utilise un catalyseur de platine, de palladium, de ruthénium, de rhodium, éventuellement appliqué sur un support inerte, un catalyseur de Raney ou un catalyseur d'oxyde métallique.

9. Procédé selon la revendication 1, 2, 3 ou 8, caractérisé en ce que le catalyseur est utilisé dans des quantités de 1 à 50 % en poids, rapportés au benzylester de l'acide (1-cyanocyclohexyl)acétique.

10. Procédé selon la revendication 1, 2, 3, 8 ou 9, caractérisé en ce que l'hydrogénation s'effectue à une pression entre 1 et 100 bars et à des températures entre 0 et 100°C.

11. Procédé selon la revendication 1, 2, 3, 8, 9 ou 10, caractérisé en ce que l'hydrogénation s'effectue en présence d'un solvant.

12. Procédé selon la revendication 2, caractérisé en ce qu'en tant qu'acide minéral on utilise du chlorure d'hydrogène, du bromure d'hydrogène ou de l'acide sulfurique anhydre.

13. Procédé selon la revendication 2 ou 12, caractérisé en ce que l'on travaille à une pression entre 1 et 10 bars et à des températures entre -20 et 50°C.

14. Procédé selon la revendication 2, 12 ou 13, caractérisé en ce que l'on travaille en présence d'un solvant.

15. Procédé selon la revendication 2, 12, 13 ou 14, caractérisé en ce que l'hydrolyse s'effectue avec l'eau à des températures entre -20 et 100°C.

16. Procédé selon la revendication 3, caractérisé en ce qu'en tant qu'acide minéral, on utilise du chlorure d'hydrogène, du bromure d'hydrogène ou de l'acide sulfurique anhydre.

17. Procédé selon la revendication 3 ou 16, caractérisé en ce que l'on travaille à une pression entre 1 et 10 bars et à des températures entre -20 et 50°C.

18. Procédé selon la revendication 3, 16 ou 17, caractérisé en ce qu'en tant qu'alcool aliphatique inférieur on utilise du méthanol ou de l'éthanol.

19. Procédé selon la revendication 3, 16, 17 ou 18, caractérisé en ce que l'on travaille en présence d'un solvant.

20. Procédé selon la revendication 3, 16, 17, 18 ou 19, caractérisé en ce que l'hydrolyse s'effectue avec de l'eau, à des températures entre -20 et 100°C.

21. Ester de l'acide (1-cyanocyclohexyl)acétique de la formule générale 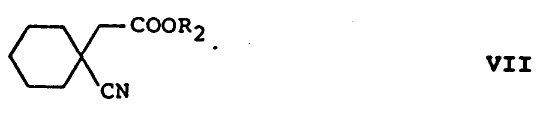 dans laquelle R₂ = alkyle avec 1 jusqu'à 4 atomes C ou un reste de benzyle de la formule générale 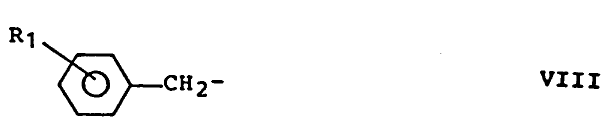 dans laquelle R₁ = H signifie un groupe alcoxy, un groupe nitro ou un halogène.

## Revendications (Revendications pour l'(les) Etat(s) contractant(s) suivant(s): ES)

1. Procédé pour la préparation de l'acide acétique de 1-(aminométhyl)cyclohexane de la formule 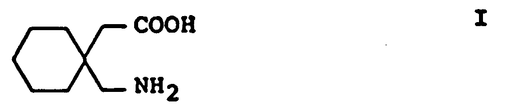 caractérisé en ce que, dans la première étape, un dialkylester de l'acide (1-cyanocyclohexyl)malonique de la formule générale 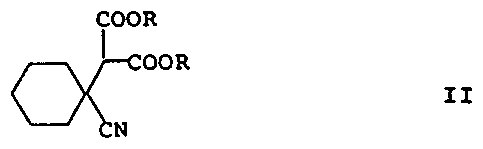 dans laquelle R signifie alkyle avec 1 jusqu'à 4 atomes de carbone, est décarbalcoxylé en l'alkylester de l'acide (1-cyanocyclohexyl)acétique correspondant de la formule générale III, 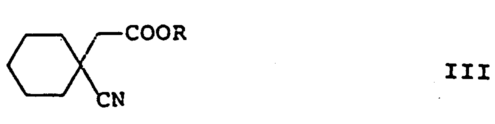 dans laquelle R a la signification indiquée, puis est transestérifié dans la deuxième étape avec un alcool de benzyle de la formule générale 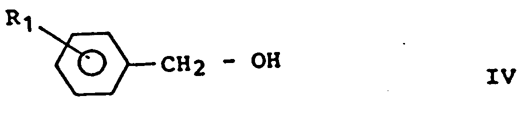 dans laquelle R₁ = H signifie un groupe alcoxy, un groupe nitro ou un halogène, en présence d'un catalyseur basique en un benzylester de l'acide (1-cyanocyclohexyl)acétique de la formule générale 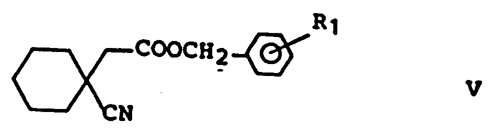 dans laquelle R₁ a la signification indiquée, puis il est hydrogéné en présence d'un catalyseur d'hydrogénation avec de l'hydrogène pour donner le produit final.

2. Procédé pour la préparation de l'acide acétique 1-(aminométhyl)cyclohexane de la formule 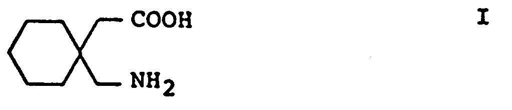 caractérisé en ce que le (1-cyanocyclohexyl)acétonitrile de la formule 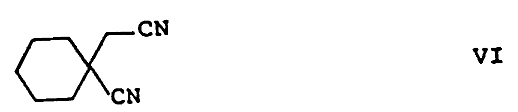 est mis en réaction avec un alcool de benzyle de la formule générale 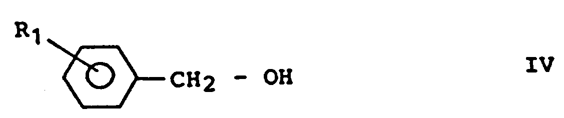 dans laquelle R₁ a la signification indiquée, en présence d'un acide minéral, et le produit réactionnel est ensuite hydrolysé avec de l'eau, après quoi le benzylester de l'acide (1-cyanocyclohexyl)acétique obtenu est hydrogéné en présence d'un catalyseur d'hydrogénation avec de l'hydrogène pour donner le produit final.

3. Procédé pour la préparation de l'acide acétique de 1-(aminométhyl)cyclohexane de la formule 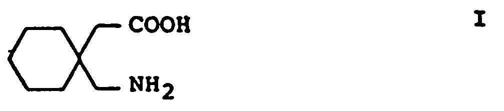 caractérisé en ce que le (1-cyanocyclohexyl)acétonitrile de la formule 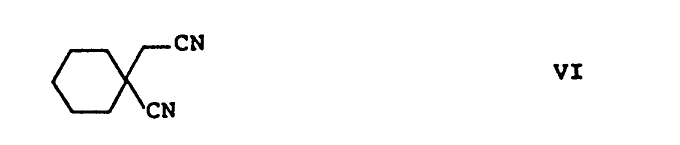 est mis en réaction avec un alcool aliphatique en C1-C4, en présence d'un acide minéral, le produit réactionnel obtenu est ensuite hydrolysé avec de l'eau, après quoi l'alkylester obtenu de l'acide (1-cyanocyclohexyl)acétique est hydrogéné en présence d'un catalyseur d'hydrogénation avec de l'hydrogène pour donner le produit final.

4. Procédé selon la revendication 1, caractérisé en ce que la décarbalcoxylation est effectuée à des températures entre 100 et 250°C, en présence d'anhydride de l'acide borique ou de solvants aprotiques dipolaires en combinaison avec de l'eau.

5. Procédé selon la revendication 4, caractérisé en ce que l'on travaille en présence de sels alcalins ou alcalino-terreux.

6. Procédé selon la revendication 1, caractérisé en ce qu'en tant que catalyseur basique pour la transestérification, on utilise un cyanure alcalin, un alcoolat alcalin ou une amine tertiaire dans une quantité de 0,01 jusqu'à 10 % en modes.

7. Procédé selon la revendication 1 ou 6, caractérisé en ce que la transestérification s'effectue en présence d'un solvant à des températures entre 0°C et la température d'ébullition de l'alcool de benzyle mis en oeuvre.

8. Procédé selon la revendication 1, 2 ou 3, caractérisé en ce qu'en tant que catalyseur d'hydrogénation, on utilise un catalyseur de platine, de palladium, de ruthénium, de rhodium, éventuellement appliqué sur un support inerte, un catalyseur de Raney ou un catalyseur d'oxyde métallique.

9. Procédé selon la revendication 1, 2, 3 ou 8, caractérisé en ce que le catalyseur est utilisé dans des quantités de 1 à 50 % en poids, rapportés au benzylester de l'acide (1-cyanocyclohexyl)acétique.

10. Procédé selon la revendication 1, 2, 3, 8 ou 9, caractérisé en ce que l'hydrogénation s'effectue à une pression entre 1 et 100 bars et à des températures entre 0 et 100°C.

11. Procédé selon la revendication 1, 2, 3, 8, 9 ou 10, caractérisé en ce que l'hydrogénation s'effectue en présence d'un solvant.

12. Procédé selon la revendication 2, caractérisé en ce qu'en tant qu'acide minéral on utilise du chlorure d'hydrogène, du bromure d'hydrogène ou de l'acide sulfurique anhydre.

13. Procédé selon la revendication 2 ou 12, caractérisé en ce que l'on travaille à une pression entre 1 et 10 bars et à des températures entre -20 et 50°C.

14. Procédé selon la revendication 2, 12 ou 13, caractérisé en ce que l'on travaille en présence d'un solvant.

15. Procédé selon la revendication 2, 12, 13 ou 14, caractérisé en ce que l'hydrolyse s'effectue avec l'eau à des températures entre -20 et 100°C.

16. Procédé selon la revendication 3, caractérisé en ce qu'en tant qu'acide minéral, on utilise du chlorure d'hydrogène, du bromure d'hydrogène ou de l'acide sulfurique anhydre.

17. Procédé selon la revendication 3 ou 16, caractérisé en ce que l'on travaille à une pression entre 1 et 10 bars et à des températures entre -20 et 50°C.

18. Procédé selon la revendication 3, 16 ou 17, caractérisé en ce qu'en tant qu'alcool aliphatique inférieur on utilise du méthanol ou de l'éthanol.

19. Procédé selon la revendication 3, 16, 17 ou 18, caractérisé en ce que l'on travaille en présence d'un solvant.

20. Procédé selon la revendication 3, 16, 17, 18 ou 19, caractérisé en ce que l'hydrolyse s'effectue avec de l'eau, à des températures entre -20 et 100°C.
